Europäisches Patentamt

European Patent Office

Office européen des brevets

(11) Publication number: 0 382 777 B1

# EUROPEAN PATENT SPECIFICATION

(45) Date of publication of patent specification: **12.10.94**   (51) Int. Cl.⁵: **A61K 9/66**, A61K 31/685, A61K 37/22, B01J 13/02

(21) Application number: **88909912.3**

(22) Date of filing: **19.10.88**

(86) International application number:
**PCT/US88/03753**

(87) International publication number:
**WO 89/03679 (05.05.89 89/10)**

(54) **AQUEOUS PREPARATION OF LIPOSOME COMPOSITION.**

(30) Priority: **19.10.87 US 110286**

(43) Date of publication of application:
**22.08.90 Bulletin 90/34**

(45) Publication of the grant of the patent:
**12.10.94 Bulletin 94/41**

(84) Designated Contracting States:
**AT BE CH DE FR GB IT LI LU NL SE**

(56) References cited:
**EP-A- 0 211 647**
**DE-A- 3 327 645**
**US-A- 4 016 100**
**US-A- 4 721 612**
**US-A- 4 731 210**

**Biochemistry, vol. 24, n 7 published 1985, Lai et al "Effects of replacement of the hydroxyl group of cholesterol and tocopherol on the thermotropic behavior of phospholipid membranes", pages 1646-1653**

(73) Proprietor: **THE LIPOSOME COMPANY, INC.**
**One Research Way**
**Princeton Forrestal Center**
**Princeton, NJ 08540 (US)**

(72) Inventor: **SWENSON, Christine, E.**
**14 Reed Drive North**
**Princeton Junction, NJ 08550 (US)**
Inventor: **MINCHEY, Sharma, R.**
**4031 Bayberry Court**
**Monmouth Junction, NJ 08852 (US)**

(74) Representative: **Warcoin, Jacques et al**
**Cabinet Régimbeau,**
**26, avenue Kléber**
**F-75116 Paris (FR)**

## Description

### Background of the Invention

The present invention involves a method of preparing a liposome composition comprising dispersing a sensitive lipid, in the hydrogen form, in an aqueous medium, absent organic solvent, at a pH at least about equal to the pK of the sensitive lipid, forming liposomes and, in one embodiment, incorporating at least one therapeutic agent into the liposomes, the composition and associated method of treatment, and a method of preparing sensitive lipid salts.

Liposomes are completely closed lipid bilayer membranes containing an entrapped aqueous volume. Liposomes may be unilamellar vesicles (possessing a single bilayer membrane ) or multilameller vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 12:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles.

Unilamellar vesicles may be produced using an extrusion apparatus by a method described in Cullis et al., PCT Application No. WO 87/00238, published January 16, 1986, entitled "Extrusion Technique for Producing Unilamellar Vesicles" incorporated herein by reference. Vesicles made by this technique, called LUVETS, are extruded under pressure through a membrane filter.

Another class of liposomes are those characterized as having substantially equal intralamellar solute distribution. This class of liposomes is denominated as stable plurilamellar vesicles as defined in U.S. Patent No. 4,522,803 to Lenk, et al., monophasic vesicles as described in U.S. Patent No. 4558,579 to Fountain, et al. and frozen and thawed multilamellar vesicles (FATMLV) wherein the vesicles are exposed to at least one freeze and thaw cycle; this procedure is described in Bally et al., PCT Publication No. 87/00043, January 15, 1987, entitled "Multilamellar Liposomes Having Improved Trapping Efficiencies".

A variety of sterols and their water soluble derivatives have been used to form liposomes; see specifically Janoff et al., PCT Publication No. WO 85/04578, published October 24, 1985, entitled "Steroidal Liposomes." Mayhew et al., PCT Publication No. WO 85/00968, published March 14, 1985, described a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., PCT Publication No. WO 87/02219, published April 23, 1987, entitled "Alpha Tocopherol-Based Vesicles."

US 4,721,612 discloses method and preparation of lipids vesicles utilizing the preformed salt form of an organic acid derivative of a sterol

The preparation of liposomes incorporating therapeutic agents generally requires organic solvents to be utilized if the preparation is to be of commercial practicality. A method of preparation that does not require organic solvents is less costly, less environmentally dangerous, and pharmaceutically advantageous.

This invention relates to the preparation of liposomes and liposomes in combination with therapeutic agents wherein the preparation is in an aqueous medium in the absence of organic solvents.

### Summary of the Invention

The instant invention provides a method of preparing a liposome composition such as claimed in claims 1 to 17 comprising dispersing a PH sensitive lipid, in the hydrogen form, in an aqueous medium, absent organic solvent, at a pH at least about equal to or above to the pK of the PH sensitive lipid, wherein the PH sensitive lipid is an organic acid derivative of a sterol or organic acid derivative of a tocopherol and wherein less than 7% (w/w) of the aqueous phase is organic solvent.

This invention includes a method of preparing a liposome composition by dispersing a PH sensitive lipid in the hydrogen form in an aqueous medium, absent organic solvent, at a pH at least about equal to the pK of the PH sensitive lipid forming liposomes and further includes, incorporating at least one therapeutic agent into the liposomes. This method further includes preferably combining lipid and solvent at a pH of at least about that which yields 75% ionization and more preferably least about that which yields 90% ionization of the lipid as predicted by the Henderson-Hasselbalch Equation. The method also includes PH sensitive lipid being the hydrogen form of a dicarboxcylic acid ester such as cholesterol hemisuccinate or tocopherol hemisuccinate.

Further included is the method wherein the therapeutic agent is, for example, an anti-infective agent, anti-inflammatory agent, keratolytic agent, anti-acne agent, anesthetic, hemorrhoidal preparation, anti-alopecia agent, antipruretic agent, antiallergy agent, antineoplastic agent, ectoparasiticidic agent, cholinomimetic agent, or cosmetic.

Specific therapeutic agents of the method are imidazoles which includes analogues and derivatives thereof.

This invention also includes a method of preparing PH sensitive lipid ionically associated with a monovalent positively charged counterion, by the process of combining in a aqueous medium, absent organic solvent, a PH sensitive lipid in the hydrogen form and a monovalent positively charged counterion and, mixing the aqueous medium of lipid and counterion. This method may further comprise dispersing the lipid in the hydrogen form by application of energy such as mechanical shearing or heating, and yet further comprise adjusting the pH of the aqueous medium to a pH in excess of the pK of the lipid.

Particularly included is the method wherein the lipid in the hydrogen form is a dicarboxylic acid ester such as cholesterol hemisuccinate ("CHS") or tocopherol hemisuccinate ("THS"). In some embodiments of this method the CHS or THS is dispersed by application of mechanical shearing and heating; and/or the monovalent positively charged counterion is sodium or tris-(hydroxymethyl)aminomethane; and/or and the aqueous medium is water.

The practice of this method for CHS and THS also comprises adjusting the pH of the aqueous medium to a pH in excess of about 5.5, and further in excess of about 7.

The invention further includes a method of preparing a pharmaceutical composition in the absence of organic solvent comprising at least one therapeutic agent, and a salt of CHS or THS, said salt being CHS or THS ionically associated with a monovalent positively charged counterion, the composition being in the form of a liposome, by the process of combining in an aqueous medium CHS or THS in the hydrogen form and a counter ion and the imidazole and mixing the CHS or THS and counterion and imidazole in the aqueous medium, in the absence of organic solvent. Various embodiments further comprise dispersing the CHS or THS by application of energy such as mechanical shearing or heating; and/or the aqueous medium being water; and/or the monovalent positively charged counterion being tris(hydroxymethyl)aminomethane or sodium; and/or adjusting the pH of the aqueous medium to a pH in excess of about 5.5 or in excess of about 7.

In the practice of this method the imidazole may be miconazole, particularly miconazole base.

The invention further provides a method of treating by sustained release of a therapeutic agent, an animal, including a human, by administering to the animal a therapeutically effective amount of a liposome preparation comprising liposomes of a monovalent positively charged salt of CHS, absent organic solvent, and liposomally encapsulated therapeutic agent, particularly where the therapeutic agent is an anti-infective.

This invention additionally includes preparing a sustained release liposome preparation comprising liposomes of a monovalent positively charged salt of CHS, absent organic solvent, and at least one liposomally encapsulated therapeutic agent. In one embodiment the therapeutic agent is an anti-infective.

Detailed Description of the Invention

In the present invention, the term lipid as used herein shall mean any suitable material resulting in a bilayer such that a hydrophobic portion of the lipid material orients toward the interior of the bilayer while a hydrophilic portion orients toward an aqueous phase. Lipids further include highly hydrophobic compounds such as triglycerides, sterols such as cholesterol which can be incorporated into a bilayer. Lipid also includes other steroid components such as polyethylene glycol derivatives of cholesterol, coprostanol, cholestanol, or cholestane, and combinations of phosphatidylcholine and cholesterol. Particularly included are organic acid derivatives of sterols (and most particularly dicarboxcyclic acid esters) such as cholesterol hemisuccinate ("CHS"). Organic acid derivatives (and most particularly dicarboxcyclic acid esters) of tocopherols may also be used as liposome-forming ingredients, such as alpha-tocopherol hemisuccinate ("THS"). Both CHS- and THS-containing liposomes and the tris(hydroxymethyl)-aminomethane ("tris") salt forms have previously been prepared by methods known in the art for preparing liposomes containing these sterols. The liposomes may also contain glycolipids.

As used herein "PH sensitive lipid" shall be understood to refer to a lipid that is morphologically sensitive to pH in being substantially increased in propensity to bilayer organization when about 90% ionized as predicted by the Henderson-Hasselbalch Equation as compared to about 10% ionized as predicted by the Henderson-Hasselbalch Equation. It is a critical limitation of this invention that the salt form of the PH sensitive lipid is prepared in aqueous medium in the absence of organic solvent. The PH sensitive lipids from which the salts are prepared are in the hydrogen ion form.

"Therapeutic agents" shall be understood to include biologically active agents ("bioactive agents") as well as other medically useful agents such as cosmetics (e.g., skin moisturizers) as well as contrast materials (e.g., dyes) and diagnostic materials.

Exemplary of bioactive agents are antifungal agents, antibacterial agents, and antiviral agents (collectively "anti-infectives"), such as imidazoles, cephalosporins (such as cephapirin), macrolides, polyenes (such as amphotericin B), aminoglycosides, magainins, tolnaftate, ciclopiroxolamine, tetracycline, lidane, thiabendazole, and bacitracin; anti-inflammatory agents; anti-alopecia agents including minoxidil; keratolytic agents; anti-acne agents including 13-cis-retinoic acid; anesthetics; hemorrhoidal preparations; antipruretic agents; anti-allergy agents; antineoplastic agents; ectoparasiticidic agents; cholinometric agents (such as pilocarpine); or cosmetics.

Imidazole will be understood to refer to imidazoles including, without limitation, miconazole, terconazole, biconazole, ketaconazole, econazole, clotrimazole and metronidazole as well as analogs and derivatives thereof characterized in having anti-infective properties.

Lipids, themselves, in particular applications may be therapeutic agents.

The pharmaceutical compositions and methods described herein have a wide range of use in treating animals including humans. Such pharmaceutical composition and methods are particularly useful in those conditions where topical application of a pharmaceutical composition is indicated.

Topical application will be understood to refer to all external applications of a medicament including oral, rectal, vaginal and ophthalmic applications. Topical application is made by any suitable method including cream, ointment, spray, and suppository.

The topical pharmaceutical compositions of this invention offer therapeutic effect with reduced potential for dispersal from the site of application. Such pharmaceutical compositions offer the particular advantage of sustained release of therapeutic agent in the therapeutically effective treatment of vaginal conditions by limited leakage from the site of application when used. For topical use preferred cream preparations of this invention are of generally uniform viscosity at both room (about 20° to 30°C) and body temperatures.

Therapeutically effective amounts of therapeutic agent as used herein will mean that amount of therapeutic agent that produces the desired effect. This amount will be understood to vary with the particular therapeutic agent, the condition being treated, the site, manner, and duration of administration and other considerations known to those skilled in the art.

Sustained release will be understood to mean a release of therapeutic agent to an animal being treated such that the duration of action of therapeutic agent is extended over that achieved by conventional delivery (see, Remington's Pharmaceutical Science, 17th ed., 1985, p. 1645).

The liposomes of this invention are prepared in the absence of organic solvent. "In the absence of" or "absent" organic solvent shall be understood to mean that substantially all liposome formation occurs in the aqueous phase, wherein any organic solvent present is not the primary solvent. An aqueous solvent is substantially absent organic solvent if the organic solvent represents less than about 7% (w/w) of the aqueous phase and preferably less than about 5%, and more preferably less than about 1% or less than about 0.5%.

The preparation of liposomes without use of organic solvents utilizes an aqueous medium, preferably water, though aqueous monovalent positively charged salt solutions of water such as buffered water, or water containing organic acids or bases (yet maintaining proper pH) are also included. In the use of salt solutions it is noted that ionic strength affects the size of liposomes produced as well as the viscosity of the liposome preparation. When salts are employed it is a limitation of the invention that such salts are those of monovalent positively charged compounds.

If acidification of the final product is desired this is usually accomplished by adding a hydrogen ion source such as an organic acid. In the preferred process, wherein the therapeutic agent is an imidazole, up to about an equimolar amount (relative to imidazole base) of organic acid is added to the preparation for acidification. A ratio of about 0.5:1 mole organic acid/imidazole base or less is preferred. (L+) lactic acid is a preferred organic acid.

In the practice of this invention, preparations may be made without organic solvents. To utilize such process sensitive lipids such as the preferred dicarboxylic acid esters CHS or THS require that a charge be established on an otherwise uncharged molecule. The presence of charge promotes bilayer organization and hence liposome formation. PH Sensitive lipids such as CHS carry a charge only when the pH of the environment is above the pK of the lipid. CHS, with a pK of about 5.5 converts from the uncharged form to the charged form as the pH of the environment approaches and exceeds 5.5. For CHS a salt is made by combining CHS, in the hydrogen form, and a monovalent positively charged counterion in an aqueous solvent at a pH of at least about 5.5 and preferably at least about 7 and most preferably at least about 8. At such pH CHS is largely deprotonated which is necessary for CHS to combine with the counterion. Deprotonation is conveniently accomplished by alkalinizing the aqueous solvent phase to a preferred pH of about 7 or 8. The amount of base required to accomplish this is calculated by the Henderson-Hasselbalch Equation which relates pH to pK and ionized to unionized molecular forms in an aqueous environment. This equation is stated:

$$pH = pK + \log\left(\frac{[A-]}{[HA]}\right)$$

where pH is the negative log of the concentration of hydrogen ion, pK is the ionization constant, and HA and A- represent the conjugate acid and base of the compound, respectively.

Increasing the pH is accomplished by a number of methods such as by adding a base such as sodium hydroxide to the aqueous solvent phase. Any suitable counterion including tris and sodium may be used. To facilitate salt formation, and due to the high hydration energy of CHS particles, it is important that CHS be dispersed in the aqueous medium. With CHS powder as a starting material dispersion is conveniently accomplished by mechanical shearing such as stirring or homogenizing accompanied by heating. In general these considerations apply to PH sensitive lipids as a class.

After addition of the counterion, lipid, in the hydrogen form, and aqueous phase are mixed thus forming the salt, such as $CHS_{tris}$ or $CHS_{sodium}$. A suitable aqueous phase in such preparation is water. Then the liposomes are prepared by adding therapeutic agent such as imidazole in dry form to the CHS salt in the aqueous solvent. The preparation is then a suspension of therapeutic agent in a liposomal cream base.

In preparing liposomes in the absence of organic solvent, an aqueous phase and a lipid mixture are conveniently heated and stirred to promote liposome formation. This is not a pressure critical step and atmospheric pressure is suitable.

Liposome preparations of this invention exhibit a sustained release in topical applications. In one embodiment a PH sensitive lipid such as CHS, in the hydrogen form, and sodium are mixed with water to form liposomes of the salt which is then dried to a powder. The resulting powder is admixed with a therapeutic agent such as erythromycin and water merely by shaking to yield a liposome composition encapsulating a therapeutic agent. The attendant ease of reconstitution of the liposome composition in dry form is of particular advantage with aqueous unstable therapeutic agents such as erythromycin in that constitution of the liposomes and therapeutic agent may be accomplished just prior to use.

A topical aqueous cream is prepared by adding lipid to the aqueous component that will comprise the final preparation. Depending on the method of preparation creams may be basic or acidic. In the case of $CHS_{sodium}$, the monovalent positively charged counterion being added is conveniently from sodium hydroxide, so the resulting cream is, without adjustment, basic. If not, any pharmaceutically acceptable agent such as an ethanolamine (e.g. tris(hydroxymethyl)aminomethane), sodium carbonate, sodium bicarbonate, sodium hydroxide and the like may be used to alkalinize the cream.

If an acidic final preparation is preferred, and the characteristics of the lipid permit, an acidic-aqueous component may be used. Organic acids such as citric acid, benzoic acid, acetic acid, and lactic acid are typical acidifying agents. For vaginal creams with lactic acid the amount of lactic acid added will depend on the desired cream composition. A preferred amount is that which results in a final pH of between about 4.0 and 7.5. However, some creams such as $CHS_{tris}$ or $THS_{tris}$ may show a phase transition at low pH (for $CHS_{tris}$ and $THS_{tris}$, at about 6.5 or below). Phase transition in THS salts may be reduced or prevented by the admixture with surfactants such as polysorbate or polyoxyl-40-stearate, usually at from about 5 to 20% (v/v) to the final composition.

Using the lipid salts and the method of preparation described above liposomes may be prepared by adding a therapeutic agent, e.g. imidazole, directly to the aqueous mixture. Miconazole, particularly in the form of miconazole base, is useful in such preparation.

Lipids of this aqueous preparation, if desired, are admixed with substances that modify the behavior of the lipid. In the preferred preparation, substances believed to be anti-oxidants such as alpha-tocopherol or butylated hydroxytoluene ("BHT") or benzyl alcohol are useful. When D-L-alpha-tocopherol is used as an anti-oxidant, a ratio of about 2 mg/gm of lipid is useful though any desired amount may be employed.

Liposome constituents may be added throughout the preparation of the composition. When adding therapeutic agents it is important to consider the physical characteristics of the agent. If the therapeutic agent is heat or pH labile, such as is the case with cephapirin, the agent is added after liposome preparation (which may require unacceptable heating) and pH adjustment. Basic therapeutic agents may be added prior to liposome formation. In some embodiment constituents such as preservatives (e.g. benzyl alcohol) or anti-oxidants or particular salts may be added during or after liposomal formation.

For imidazoles, topical liposomal cream preparations preferably contain up to about 400 mg imidazole/g cream for vaginal use, but this is variably at the discretion of the medical profession and the characteristics of the particular imidazole. In view of the toxicity of many imidazoles, it is an important consideration of such topical preparations that only limited amounts of the imidazole enter the subject animal. The site of action in such topical uses is the external infective agent being treated. Miconazole/CHS$_{salt}$ cream for vaginal use is preferred at a strength of about 150 to 250 mg miconazole/g final cream preparation with about 200 mg/g (20%) being most preferred when application is to be in a single dose for vaginal candidiasis. The miconazole/CHS$_{salt}$ cream is stable at concentrations up to about 20% by weight.

Liposomal cream preparations preferably contain up to about 200 mg terconazole/g cream for vaginal use, but this is variably at the discretion of the medical professional. Terconazole cream for vaginal use is preferred at a strength of about 5 to 100 mg terconazole/g final cream preparation with about 20 mg/g (2%) being most preferred when application is to be in a single dose for vaginal candidiasis. The miconazole cream is stable at concentrations up to about 20% by weight. For vaginal dosage in humans an application of about 5 gm of a 20 mg/gm preparation is useful. Animal dosages tend to be approximately proportional to human dosages relative to body weight of the animal.

The efficiency of the liposomal preparations was tested against a currently available imidazole preparation of a 2% miconazole-nitrate cream (Monistat™, Ortho Pharmaceuticals). For this test a rat model of Candida albicans was used.

Ovariectomized rats were injected subcutaneously with 0.5 mg of 17-$\beta$-estradiol valerate in sesame oil at weekly intervals to produce a state of constant estrus. On day zero, rats were inoculated intravaginally with approximately 10 colony forming units (CFU) of C. albicans in 0.1 ml saline. On day 3, the vaginas of all rats were swabbed and cultured for Candida to confirm the presence of an infection. Immediately after swabbing, the rats were treated with 0.2 ml of the liposomal test formulation intravaginally, while 2% miconazole-nitrate cream treated rats received 0.2 ml of 2% miconazole nitrate cream 2 times per day for 3 days. The liposomal test formulation was cream of CHS$_{tris}$ with 200 mg miconazole/gm of cream. On day 10, the vaginas of all rats with greater than 100 CFU of Candida per vaginal swab on day 3 were swabbed and cultured again to determine if the treatment was successful. On day 10, all untreated rats showed greater than 300 CFU of Candida per vaginal swab. Rats with less than 25 CFU were considered "cured" and those with less than 100 CFU were considered "improved".

In a test model imidazole liposome preparations in a single application proved to be the equivalent of 3 days of treatment with twice daily miconazole-nitrate cream. Thus, liposomal imidazole as prepared by the instant aqueous method in the absence of organic solvent yields an effective composition exhibiting a sustained release of therapeutic agent with ease of administration of therapeutic agent such as imidazole. Also useful on the treatment of such animals is the administration of liposomal preparations of this invention utilizing terconazole.

Example 1

Preparation of a Salt of CHS

A salt of CHS was prepared by combining 16 gm of powdered CHS in the hydrogen form and 1.32 gm of sodium hydroxide with 20 cc of distilled water. The CHS powder was thoroughly dispersed in water by vigorous mixing with a mechanical stirrer accompanied by heating to 75°C. This process continued for 2 hours. The resulting mixture contained CHS$_{sodium}$ in the form of liposomes.

Example 2

Preparation of a Salt of THS

A salt of THS was prepared by combining 1 gm of powdered THS in the hydrogen form and 0.074 gm of sodium hydroxide with 10 cc of distilled water. The THS powder was thoroughly dispersed in water by vigorous mixing with an homogenizer (Polytron™, Brinkman, Westbury, N.Y.) while avoiding excessive heating by use of an ice bath. This process continued for 5 minutes. The pH of the resulting mixture was adjusted to approximately 7.0. The resulting mixture contained THS$_{sodium}$ in the form of liposomes.

Example 3

Aqueous Preparation of Miconazole-CHS$_{(tris)}$ Cream

To prepare a cream, 50 grams of miconazole-CHS$_{tris}$ containing 200 mg/ml (20%) miconazole base was prepared by first preparing a CHS salt. To begin 3.6 gm of tris buffer (free base) and 0.05 gm NaOH was dissolved in 19.55 ml of water. The solution was then heated to 80°C and combined with 14.0 gm of powdered CHS in the hydrogen form and 0.05 gm of alpha-tocopherol with stirring thus forming CHS$_{tris}$ in the form of liposomes.

To prepare the cream the CHS plus tris mixture was further heated and stirred until no CHS crystals were visible at 100X magnification. Next, the mixture was cooled to room temperature (20° to 30°C) and 10.0 gm of miconazole was then added with stirring. In this preferred preparation 0.125 gm of the preservative benzyl alcohol was then added with stirring. The pH was then adjusted to 7.0-8.0 with lactic acid. This required about 0.625 ml of 30% (w/v) aqueous L(+) lactic acid solution. Finally, water was added to bring the final weight up to 50 gm.

Example 4

Aqueous Preparation of Miconazole-CHS$_{sodium}$Cream

To prepare 50 gm of a cream of miconazole in association with the sodium salt of CHS ("CHS$_{sodium}$") containing 200 mg/ml (20%) miconazole, was prepared by general procedure of Example 3 was followed. First, 1.32 gm NaOH was dissolved in 20 ml water. Then the mixture was heated to 80°C and combined with 16.0 gm of CHS, in the hydrogen form, and 0.05 gm D-L-alpha-tocopherol while stirring, thus forming CHS$_{sodium}$. To this was added 10g miconazole base, 0.25 gm benzyl alcohol and the pH was adjusted with about 0.7 ml of 30% (w/v) aqueous L-(+) lactic acid solution. Finally, water was added to bring the final weight of the resulting cream up to 50gm.

**Claims**

1. A method of preparing a liposome comprising the salt form of a pH sensitive lipid wherein the method comprises the step of preparing the salt form of the pH sensitive lipid by dispersing the pH sensitive lipid in its hydrogen form in an aqueous medium in the absence of an organic solvent at a pH at least equal to or above the pK of the pH sensitive lipid ; wherein the pH sensitive lipid is an organic acid derivative of a sterol or organic acid derivative of a tocopherol and wherein less than 7% (w/w) of the aqueous phase is organic solvent.

2. The method of claim 1 wherein less than 5% (w/w) of the aqueous phase is organic solvent, preferably wherein less than 1% (w/w) of the aqueous phase is organic solvent and even more preferably wherein less than 0,5% (w/w) of the aqueous phase is organic solvent.

3. The method of claim 2 further comprising incorporating at least one therapeutic agent into the liposomes.

4. The method of claim 3 wherein the therapeutic agent is an anti-infective agent, anti-inflammatory agent, keratolytic agent, anti-acne agent, anesthetic, hemorrhoidal preparation, anti-alopecia agent, anti-pruretic agent anti-allergy agent, antineoplastic agent, ectoparasiticidic agent, cholinomimetic agent, or cosmetic.

5. The method of claim 4 wherein the anti-infective agent is an imidazole.

6. The method of claim 5 wherein the imidazole is miconazole.

7. The method of any one of claims 1 to 6 wherein the lipid is the hydrogen form of a dicarboxylic acid ester.

8. The method of claim 7 wherein the dicarboxylic acid ester is cholesterol hemisuccinate or tocopherol hemisuccinate.

9. The method of any one of claims 1 to 8 wherein step (a) further includes combining at a pH of at least about that which yields 75 % ionization of the lipid as predicted by the Henderson-Hasselbalch Equation.

10. The method of any one of claims 1 to 8 wherein step (a) further includes combining at a pH of at least about that which yields 90 % ionization of the lipid as predicted by the Henderson-Hasselbalch Equation.

11. A method according to any one of claims 1 to 10, comprising preparing a pH sensitive lipid ionically associated with a monovalent positively charged counterion, by the process of combining the pH sensitive lipid in the hydrogen form and the monovalent positively charged counterion in an aqueous medium in the absence of organic solvent.

12. The method of claim 11 further comprising combining lipid and counterion by dispersing the lipid in the hydrogen form by application of mechanical shearing and heating.

13. The method of claims 11 or 12 further comprising adjusting the pH of the aqueous medium to a pH in excess of the pK of the lipid.

14. The method of any one of claims 11 to 13 wherein the counterion is sodium or tris-

(hydroxymethyl)aminomethane.

15. The method of any one of claims 11 to 14 wherein the aqueous medium is water.

16. The method of any one of claims 11 to 15 further comprising adjusting the pH of the aqueous medium to a pH in excess of about 5.5.

17. The method of any one of claims 11 to 16 further comprising adjusting the pH to in excess of about 7.

18. A method of preparing a pharmaceutical composition in the absence of organic solvent comprising a method of preparing a liposome incorporating at least one therapeutic agent according to any one of claims 1 to 17.

19. A method of preparing a pharmaceutical composition in the absence of organic solvent according to claim 18 comprising at least one imidazole and a salt of cholesterol hemisuccinate or tocopherol hemisuccinate being cholesterol hemisuccinate or tocopherol hemisuccinate ionically associated with a monovalent positively charged counterion, the composition being in the form of a liposome by the process of combining cholesterol hemisuccinate or tocopherol hemisuccinate in the hydrogen form and the counterion and imidazole in an aqueous medium in the absence of organic solvent.

20. The method of claim 19 further comprising combining the cholesterol hemisuccinate or tocopherol hemisuccinate and counterion and imidazole by dispersing the cholesterol hemisuccinate or the tocopherol hemisuccinate in the hydrogen form by application of mechanical shearing and heating.

**Patentansprüche**

1. Verfahren zur Herstellung eines Liposomen, der die Salzform eines pH-empfindlichen Lipides enthält, wobei das Verfahren die Stufe der Herstellung der Salzform des pH-empfindlichen Lipides unfaßt durch Dispersion des pH-empfindlichen Lipides in dessen Wasserstoffform in einem wäßrigen Medium in Abwesenheit eines oganischen Lösungsmittels bei einem pH, der wenigstens gleich oder oberhalb des pK des pH-empfindlichen Lipides liegt; wobei das pH-empfindliche Lipid ein organisches Säurederivat eines Sterols oder ein organisches Säurederivat eines Tocopherols ist, und worin weniger als 7% (w/w) der wäßrigen Phase organisches Lösungsmittel ist.

2. Verfahren nach Anspruch 1, worin weniger als 5% (w/w) der wäßrigen Phase organisches Lösungsmittel ist, vorzugsweise worin weniger als 1% (w/w) der wäßrigen Phase organisches Lösungsmittel ist und noch bevorzugter, worin weniger als 0,5% (w/w) der wäßrigen Phase organisches Lösungsmittel ist.

3. Verfahren nach Anspruch 2, das weiterhin die Einbeziehung von wenigstens einem therapeutischen Mittel in die Liposomen unfaßt.

4. Verfahren nach Anspruch 3, worin das therapeutische Mittel ein Anti-Infektionsmittel, ein entzündungswidriges Mittel, keratolytisches Mittel, Anti-Akne-Mittel, Anästhetikum, eine hämorrhoidale Präparation, ein Anti-Alopezie-Mittel, anti-pruretisches Mittel, anti-allergisches Mittel, anti-neoplastisches Mittel, ektoparasitizides Mittel, cholinomimetisches Mittel oder ein Kosmetikum ist.

5. Verfahren nach Anspruch 4, worin das Anti-Infektionsmittel ein Imidazol ist.

6. Verfahren nach Anspruch 5, worin das Imidazol Miconazol ist.

7. Verfahren nach einem der Ansprüche 1 bis 6, worin das Lipid die Wasserstofform eines Dicarbonsäureesters ist.

8. Verfahren nach Anspruch 7, worin der Dicarbonsäureester Cholesterin-hemisuccinat oder Tocopherol-hemisuccinat ist.

9. Verfahren nach einem der Ansprüche 1 bis 8, worin die Stufe (a) weiterhin umfaßt die Vereinigung bei einem pH von wenigstens etwa dem, der 75% Ionisierung des Lipids erbringt, wie durch die Henderson-Hasselbalch-Gleichung vorausberechnet.

10. Verfahren nach einem der Ansprüche 1 bis 8, worin die Stufe (a) weiterhin umfaßt die Vereinigung bei einem pH von wenigstens etwa dem, der 90% Ionisierung des Lipids erbringt, wie durch die Henderson-Hasselbalch-Gleichung vorausberechnet.

11. Verfahren nach einem der Ansprüche 1 bis 10, das die Herstellung eines pH-empfindlichen Lipids unfaßt, das ionisch assoziiert mit einem einwertig positiv geladenen Gegen-Ion ist durch das Verfahren der Vereinigung des pH-emfpindlichen Lipids in der Wasserstofform und des einwertig positiv geladenen Gegen-Ions in einem wäßrigem Medium in Abwesenheit von organischem Lösungsmittel.

12. Verfahren nach Anspruch 11, das weiterhin die Vereinigung von Lipid und Gegen-ion durch Dispersion des Lipids in der Wasserstofform unter Anwendung von mechanischem Scheren und Erhitzen unfaßt.

13. Verfahren nach Anspruch 11 oder 12, das weiterhin die Einstellung des pH des wäßrigen Mediums auf einen pH oberhalb des pK des Lipids unfaßt.

14. Verfahren nach einem der Ansprüche 11 bis 13, worin das Gegen-Ion Natrium oder Tris-(hydroxymethyl)aminomethan ist.

15. Verfahren nach einem der Ansprüche 11 bis 14, worin das wäßrige Medium Wasser ist.

16. Verfahren nach einem der Ansprüche 11 bis 15, das weiterhin die Einstellung des pH des wäßrigen Mediums auf einen pH oberhalb von etwa 5,5 umfaßt.

17. Verfahren nach einem der Ansprüche 11 bis 16, das weiterhin die Einstellung des pH oberhalb von etwa 7 unfaßt.

18. Verfahren zur Herstellung einer pharmazeutischen Zusammensetzung in Abwesenheit von organischen Lösungsmittel, das ein Verfahren zur Herstellung eines Liposomen unfaßt, der wenigstens ein therapeutisches Mittel nach einem der Ansprüche 1 bis 17 enthält.

19. Verfahren zur Herstellung einer pharmazeutischen Zusammensetzung in Abwesenheit von organischen Lösungsmittel nach Anspruch 18, das wenigstens ein Imidazol und ein Salz von Cholesterin-hemisuccinat oder Tocopherol-hemisuccinat unfaßt, welches Cholesterin-hemisuccinat oder Tocopherol-hemisuccinat ionisch assoziiert mit einem einwertig positiv geladenen Gegen-Ion ist, wobei die Zusammensetzung in Form eines Liposoms vorliegt durch das Verfahren zur Vereinigung von Cholesterin-hemisuccinat oder Tocopherol-hemisuccinat in der Wasserstofform und dem Gegen-Ion und Imidazol in einem wäßrigen Medium in Abwesenheit von organischem Lösungsmittel.

20. Verfahren nach Anspruch 19, das weiterhin die Vereinigung von Cholesterin-hemisuccinat oder Tocopherol-hemisuccinat und dem Gegen-Ion und Imidazol umfaßt durch Dispergieren des Cholesterin-hemisuccinates oder des Tocopherol-hemisuccinates in der Wasserstofform durch Anwenden mechanischer Scherkräfte und Erhitzen.

**Revendications**

1. Procédé pour la préparation d'un liposome comprenant la forme sel d'un lipide sensible au pH, dans lequel le procédé comprend l'étape de préparation de la forme sel du lipide sensible au pH par dispersion du lipide sensible au pH sous sa forme hydrogène dans un milieu aqueux en l'absence de solvant organique à un pH au moins égal ou supérieur au pK du lipide sensible au pH; le lipide sensible au pH étant un dérivé d'acide organique d'un stérol ou un dérivé d'acide organique d'un tocophérol et moins de 7% (en poids/poids) de la phase aqueuse étant un solvant organique.

2. Procédé selon la revendication 1, dans lequel moins de 5% (en poids/poids) de la phase aqueuse est un solvant organique, de préférence dans lequel moins de 1% (en poids/poids) de la phase aqueuse est un solvant organique et plus avantageusement encore dans lequel moins de 0,5% (en poids/poids) de la phase aqueuse est un solvant organique.

3. Procédé selon la revendication 2, comprenant en outre l'incorporation d'au moins un agent thérapeutique dans les liposomes.

4. Procédé selon la revendication 3, dans lequel l'agent thérapeutique est un agent anti-infectieux, un agent anti-inflammatoire, un agent kératolytique, un agent anti-acné, un anesthésique, une préparation hémorrhoïdaire, un agent anti-alopécie, un agent antiprurigineux, un agent anti-allergique, un agent antinéoplasique, un agent ectoparasiticide, un agent cholinomimétique ou un cosmétique.

5. Procédé selon la revendication 4, dans lequel l'agent anti-infectieux est un imidazole.

6. Procédé selon la revendication 5, dans lequel l'imidazole est du miconazole.

7. Procédé selon l'une quelconque des revendications 1 à 6, dans lequel le lipide est la forme hydrogène d'un ester d'acide dicarboxylique.

8. Procédé selon la revendication 7, dans lequel l'ester d'acide dicarboxylique est l'hémisuccinate de cholestérol ou l'hémisuccinate de tocophérol.

9. Procédé selon l'une quelconque des revendications 1 à 8, dans lequel l'étape (a) comprend en outre la combinaison à un pH d'au moins environ celui qui donne 75% d'ionisation du lipide comme prédit par l'équation de Henderson-Hasselbalch.

10. Procédé selon l'une quelconque des revendications 1 à 8, dans lequel l'étape (a) comprend en outre la combinaison à un pH d'au moins environ celui qui donne 90% d'ionisation du lipide comme prédit par l'équation de Henderson-Hasselbalch.

11. Procédé selon l'une quelconque des revendications 1 à 10, comprenant la préparation d'un lipide sensible au pH associé ioniquement avec un contre-ion monovalent chargé positivement, par le procédé consistant à combiner le lipide sensible au pH sous la forme hydrogène et le contre-ion monovalent chargé positivement dans un milieu aqueux en l'absence de solvant organique.

12. Procédé selon la revendication 11, comprenant en outre la combinaison d'un lipide et d'un contre-ion par dispersion du lipide sous la forme hydrogène par application d'un cisaillement mécanique et d'un chauffage.

13. Procédé selon l'une des revendications 11 ou 12, comprenant en outre l'ajustement du pH du milieu aqueux à un pH excédant le pK du lipide.

14. Procédé sellon l'une quelconque des revendications 11 à 13, dns lequel le contre-ion est du sodium ou du tris(hydroxyméthyl)-aminométhane.

15. Procédé selon l'une quelconque des revendications 11 à 14, dans lequel le milieu aqueux est de l'eau.

16. Procédé selon l'une quelconque des revendications 11 à 15, comprenant en outre l'ajustement du pH du milieu aqueux à un pH excédant environ 5,5.

17. Procédé selon l'une quelconque des revendications 11 à 16, comprenant en outre l'ajustement du pH à une valeur dépassant environ 7.

18. Procédé pour la préparation d'une composition pharmaceutique en l'absence de solvant organique, comprenant un procédé pour la préparation d'un liposome comportant au moins un agent thérapeutique selon l'une quelconque des revendications 1 à 17.

19. Procédé pour la préparation d'une composition pharmaceutique, en l'absence de solvant organique selon la revendication 18, comprenant au moins un imidazole, et un sel d'hémisuccinate de cholestérol ou d'hémisuccinate de tocophérol étant un hémisuccinate de cholestérol ou un hémisuccinate de tocophérol associé ioniquement avec un contre-ion monovalent chargé positivement, la composition étant sous la forme d'un liposome, par le procédé de combinaison d'hémisuccinate de cholestérol ou d'hémisuccinate de tocophérol sous la forme hydrogène et le contreion étant un imidazole dans un milieu aqueux en l'abence de solvant organique.

20. Procédé selon la revendication 19, comprenant en oute la combinaison de l'hémisuccinate de cholestérol ou de l'hémisuccinate de tocophérol et un contre-ion et de l'imidazole, par dispersion de l'hémisuccinate de cholestérol ou de l'hémisuccinate de tocophérol sous la forme hydrogène par application de cisaillement mécanique et de chauffage.